(19) Europäisches Patentamt

European Patent Office

Office européen des brevets

(11) **EP 2 798 334 B1**

(12) **FASCICULE DE BREVET EUROPEEN**

(45) Date de publication et mention de la délivrance du brevet:
**30.03.2016 Bulletin 2016/13**

(21) Numéro de dépôt: **12818519.6**

(22) Date de dépôt: **26.12.2012**

(51) Int Cl.:
**G01N 21/63** *(2006.01)*  **G01N 33/00** *(2006.01)*

(86) Numéro de dépôt international:
**PCT/EP2012/076898**

(87) Numéro de publication internationale:
**WO 2013/098289 (04.07.2013 Gazette 2013/27)**

(54) **DISPOSITIF DE DETECTION ET/OU DE DOSAGE D'HYDROGENE ET PROCEDE DE DETECTION ET/OU DE DOSAGE D'HYDROGENE**

VORRICHTUNG ZUM NACHWEIS UND/ODER ZUR DOSIERUNG VON WASSERSTOFF SOWIE VERFAHREN ZUM NACHWEIS UND/ODER ZUR DOSIERUNG VON WASSERSTOFF

DEVICE FOR DETECTING AND/OR DOSING HYDROGEN AND METHOD OF DETECTING AND/OR DOSING HYDROGEN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.12.2011 FR 1162587**

(43) Date de publication de la demande:
**05.11.2014 Bulletin 2014/45**

(73) Titulaire: **Agence Nationale pour la Gestion des Déchets Radioactifs**
**92298 Châtenay-Malabry (FR)**

(72) Inventeurs:
• **BERTRAND, Johan**
**F-91570 Bievres (FR)**
• **DELEPINE-LESOILLE, Sylvie**
**F-94240 l'Hay Les Roses (FR)**
• **PHERON, Xavier**
**F-42000 Saint-Etienne (FR)**

(74) Mandataire: **Brevalex**
**95, rue d'Amsterdam**
**75378 Paris Cedex 8 (FR)**

(56) Documents cités:
EP-A1- 2 362 190   WO-A1-2009/067671
WO-A1-2010/009951   WO-A1-2011/022829
JP-A- 61 017 048

• YIBING ZHANG ET AL: "Applications of optical fiber sensors in the oil refining and petrochemical industries", 2011 IEEE SENSORS PROCEEDINGS : LIMERICK, IRELAND, 28 - 31 OCTOBER 2011, IEEE, PISCATAWAY, NJ, 28 octobre 2011 (2011-10-28), pages 246-249, XP032093296, DOI: 10.1109/ICSENS.2011.6127094 ISBN: 978-1-4244-9290-9

## Description

## DOMAINE TECHNIQUE

L'invention se rapporte au domaine la détection et du dosage d'hydrogène.

Les installations, telles que les lieux de stockage, les forages géothermiques, les lieux d'entreposage et les réservoirs industriels de produits chimiques et/ou radioactifs, peuvent être soumis à des risques de dégagement d'hydrogène liés aux produits stockés, ces dégagements d'hydrogène sont explosifs et peuvent être, dans certaines conditions nocifs pour l'homme.

Pour contrôler ces risques et détecter préventivement tout dégagement d'hydrogène qui pourrait avoir lieu, il est commun d'équiper ce type de lieux de stockage de détecteurs chimiques adaptés pour la détection et/ou la mesure d'hydrogène. En raison des durées de stockage longues (plusieurs années) des produits chimiques ou radioactifs et de l'inaccessibilité des ouvrages de stockage, l'instrumentation et les détecteurs chimiques associés, tels que les détecteurs d'hydrogène, pour des raisons évidentes de sécurité, doivent être durables dans le temps, c'est-à-dire présenter une stabilité et une sensibilité optimums durant tout ou partie de la durée du stockage. Ils doivent de surcroît être déployés en grand nombre pour couvrir de grandes dimensions et caractériser des ambiances inhomogènes. Enfin, le dispositif de mesure doit prendre en compte les aspects des risques d'explosions et doit pouvoir garantir une sécurité intrinsèque du dispositif.

L'invention se rapporte donc plus spécifiquement à un dispositif de détection et/ou de dosage d'hydrogène, à un procédé de détection et/ou dosage de l'hydrogène.

## ÉTAT DE LA TECHNIQUE ANTÉRIEURE

Il est connu, lorsqu'il s'agit de surveiller la présence d'hydrogène dans des installations, telles que des lieux de stockage de produits chimiques et/ou radioactifs, présentant des zones à surveiller dont l'étendue dépasse la centaine de mètres, d'utiliser des dispositifs de détection et/ou de dosage d'hydrogène utilisant une fibre optique en tant que capteur d'hydrogène. Ce type de dispositif présente, entre autres avantages, de permettre une détection garantissant la sécurité intrinsèque de l'installation car, il utilise une mesure optique sans risque d'étincelles qui pourraient déclencher une explosion dans un milieu gazeux contenant un grande quantité d'hydrogène.

On entend ci-dessus et dans le reste de ce document par hydrogène à la fois l'hydrogène sous sa forme atomique, sous sa forme moléculaire (c'est-à-dire le dihydrogène) ou sous sa forme isotopique qu'est le deutérium.

Ainsi, le document WO 2009/067671 décrit un tel dispositif de détection et/ou de dosage d'hydrogène.

Le dispositif décrit dans le document WO 2009/067671 comporte :

- une fibre optique de mesure destinée à équiper l'installation dans la zone à surveiller,
- un système optique relié optiquement à la fibre optique et adapté pour mesurer la variation de l'absorption de la lumière par ladite fibre optique à une longueur d'onde bien définie.

Un tel dispositif utilise la propriété de diffusion d'hydrogène dans une fibre optique et la détérioration des propriétés de transmission à certaines longueurs d'onde qui s'ensuit. En effet, l'absorption d'hydrogène dans une fibre entraîne la création de groupement hydroxyle OH et dihydrogène moléculaire $H_2$ absorbant à certaines longueurs d'onde dans l'infrarouge telles qu'à 1080, 1180 et 1240 nm. L'atténuation de la transmission de la fibre à ces longueurs d'onde est donc directement liée à la concentration d'hydrogène dans celle-ci.

Ainsi, lorsqu'un tel dispositif équipe une installation à surveiller avec la fibre optique déployée sur l'ensemble de la zone à surveiller, une fuite d'hydrogène entraînera une absorption d'une partie de l'hydrogène par la fibre optique capteur qui pourra être détectée par ladite mesure d'absorption de lumière.

Néanmoins, si un tel dispositif permet une détection efficace de la présence d'hydrogène dans l'installation, elle présente un certain nombre d'inconvénients. En effet, un tel dispositif est dépendant du vieillissement de la fibre optique qui, si cet état n'est pas parfaitement connu, peut entraîner une détection erronée de la présence d'hydrogène. En effet, les vieillissements des fibres optiques induisent des pertes de transmission, usuellement inhomogènes dans la gamme spectrale du proche infrarouge, donc entraînant des incertitudes sur la détection d'hydrogène avec l'invention décrite dans WO 2009/067671.

Le document WO 2008/136870 décrit un autre type de dispositif de détection et/ou de dosage d'hydrogène pour lequel la fibre optique de mesure comprend au moins un réseau de Bragg inscrit, c'est-à-dire une portion de la fibre sur laquelle l'indice de réfraction est modulé de façon périodique, et un revêtement cible de la partie de la fibre optique qui reçoit le réseau de Bragg. Le revêtement cible est adapté pour l'adsorption d'hydrogène. Un tel dispositif comprend, de façon similaire au dispositif décrit WO 2009/067671, la fibre optique de mesure qui est destinée à équiper l'installation et un système optique relié optiquement à la fibre optique.

Le système optique, pour un tel dispositif est configuré pour émettre un rayonnement électromagnétique à différentes longueurs d'onde dans la fibre optique et pour mesurer la longueur d'onde à laquelle chaque réseau de Bragg inscrit réfléchit ledit rayonnement électromagnétique. En effet, les caractéristiques d'un réseau de Bragg inscrit sont dépendantes de la quantité d'hydrogène adsorbée par le revêtement cible. Il est donc possible, en mesurant la longueur d'onde à laquelle réfléchit l'un des réseaux de Bragg, de remonter à la quantité d'hydrogène adsorbée par le revêtement correspondant.

Ainsi, lorsqu'un tel système est mis en place dans une installation à surveiller, la fibre optique comportant le ou les réseaux de Bragg inscrits étant déployée sur l'ensemble de la zone à surveiller, une fuite d'hydrogène à proximité d'un des réseaux de Bragg inscrits entraîne un décalage dans la longueur d'onde du rayonnement électromangnétique réfléchi par ce réseau de Bragg et permet de détecter la fuite et l'endroit où elle a lieu.

Un tel dispositif permet donc une détection d'hydrogène dans l'installation au niveau de chacun des réseaux de Bragg inscrits avec, s'il y a multiplexage des réseaux de Bragg, possibilité d'identifier l'origine géographique de la fuite d'hydrogène.

Si un dispositif tel que décrit dans WO 2008/136870 permet de détecter la présence d'hydrogène dans l'installation avec une possibilité d'identification de l'origine géographique de la fuite, de dispositif présente un certain nombre d'inconvénients. En effet, la fibre optique présente un nombre d'emplacements au niveau desquels la détection et/ou le dosage est obtenu qui est limité puisque dépendant de l'inscription d'un réseau de Bragg et de la technologie de multiplexage utilisée. On peut également noter que le vieillissement des revêtements cibles, tels que celui décrit dans WO 2009/067671, n'est pas connu et présente une incertitude quant au maintien de la sensibilité du dispositif pour des durées supérieures à plusieurs années.

De plus un tel dispositif présente également des risques de disparition des réseaux de Bragg en raison des conditions d'expositions de la fibre optique à l'hydrogène.

Le document JP61017048-A décrit un dispositif de détection d'hydrogène comportant une fibre optique de mesure avec un système optique relié optiquement à la fibre et adapté pour mesurer un paramètre selon un principe de mesure du type Raman.

**EXPOSÉ DE L'INVENTION**

La présente invention vise à remédier à ces inconvénients.

Un des buts de l'invention est donc de fournir un dispositif de détection et/ou de dosage d'hydrogène mettant en oeuvre une fibre optique pour la détection d'hydrogène qui est apte, lorsqu'il équipe la zone à surveiller d'une installation, à détecter la présence d'hydrogène et d'identifier la localisation de cette source d'hydrogène ceci de manière continue sur toute la longueur de la fibre optique, un tel dispositif devant présenter une détection et/ou un dosage en hydrogène qui sont peu dépendants de l'état de vieillissement de la fibre optique vis-à-vis d'un dispositif de l'art antérieur.

A cet effet, l'invention se rapporte à un dispositif de détection et/ou de dosage d'hydrogène destiné à la surveillance d'une installation, ledit dispositif comportant :

- une première fibre optique de mesure destinée à équiper l'installation,
- un système optique relié optiquement à la première fibre optique de mesure et adapté pour mesurer au moins un paramètre de la première fibre optique,

le système optique étant adapté pour effectuer la mesure du paramètre de la première fibre optique le long de la première fibre optique de mesure selon un principe de mesure du type Brillouin.

Ainsi, un tel dispositif en utilisant le principe de la mesure Brillouin pour mesurer un paramètre de la première fibre optique de mesure permet la détection d'une variation de l'indice effectif de propagation du mode optique $n_{eff}$ le long de la première fibre optique de mesure, cette variation influençant directement les pics Brillouin et donc les paramètres mesurés lors d'une mesure selon le principe de la mesure du type Brillouin. La diffusion d'hydrogène dans une fibre optique ayant un effet sur l'indice effectif de propagation du mode optique $n_{eff}$, une telle diffusion, en entraînant la variation de l'indice de réfraction, et donc des paramètres mesurés selon le principe de la mesure du type Brillouin, est détectable et quantifiable au moyen d'un tel dispositif.

Une telle mesure dépendant de l'indice effectif de propagation du mode optique $n_{eff}$, contrairement à celle dépendant la variation de la transmission de la fibre optique à une longueur d'onde donnée, en n'étant reliée qu'à une variation du pic Brillouin ne présente pas de dépendance marquée vis-à-vis de l'état de vieillissement de la fibre optique. Un tel dispositif permet donc une détection et/ou un dosage qui ne sont pas dépendants de l'état de vieillissement de la fibre optique et qui reste donc fiable dans le temps.

Cette mesure pouvant être, selon un principe identique à la mesure de température Brillouin le long d'une fibre optique, réalisée sur toute la longueur de la première fibre optique de mesure avec une résolution spatiale inférieure au mètre sur une distance supérieure à la dizaine de kilomètres, permet de localiser l'endroit précis où a lieu la variation du paramètre et donc la portion précise de la première fibre optique de mesure dans laquelle a eu lieu la diffusion d'hydrogène.

Un tel dispositif permet donc, lorsqu'il équipe une zone à surveiller d'une installation, la détection et la quantification d'une source d'hydrogène avec une identification de l'emplacement précis de cette source dans la zone à surveiller, sans avoir à anticiper les lieux de ces événements, ceci sur des temps longs, puisque la mesure n'est que très peu impactée par le vieillissement de la fibre optique de mesure.

Par mesure selon le principe d'une mesure du type Brillouin, on entend ci-dessus et dans le reste de ce document, la mesure d'au moins un paramètre concernant (i) le spectre de rétrodiffusion Brillouin (qu'il s'agisse des fréquences Stokes ou Anti-stokes, des pics principal ou secondaires, liés aux différents modes acoustiques ayant un recouvrement non nul avec le mode optique) ou (ii) du gain Brillouin. Ce paramètre peut être par exemple, le décalage en fréquence d'un des deux pics de rétrodiffusion Brillouin par rapport à l'impulsion électromagnétique à l'origine du phénomène de rétrodiffusion Brillouin ou entre pics, l'intensité de l'un de ces deux pics Brillouin ou encore leurs formes (largeur du pic à mi-hauteur par exemple).

On entend ci-dessus et dans le reste de ce document par pic Brillouin, aussi bien un pic de rétrodiffusion Brillouin spontané qu'un pic de gain Brillouin, le type de pic dépendant directement du type de mesure Brillouin effectué par le dispositif lors de sa mise en oeuvre. Ainsi, par exemple, pour un dispositif effectuant une mesure Brillouin selon le principe de la réflectométrie.

La mesure du paramètre de la première fibre optique peut être réalisée selon un principe de mesure du type Brillouin sélectionné dans le groupe de procédés comportant la mesure de réflectométrie optique Brillouin associée à une méthode de résolution spatiale par codage dans le domaine temporel, la mesure de réflectométrie optique Brillouin associée à une méthode de localisation par codage dans le domaine fréquentiel, la mesure de réflectométrie optique Brillouin dans le domaine de corrélation, la mesure optique de gain Brillouin associé à une analyse dans le domaine temporel, la mesure optique de gain Brillouin dans le domaine fréquentiel et la mesure optique de gain Brillouin par analyse dans le domaine de la corrélation.

Ainsi, le dispositif peut être adapté en fonction des besoins liés à l'installation à surveiller aussi bien en termes de résolution spatiale, de la distance le long de laquelle est effectuée la surveillance, le seuil de détection d'hydrogène et la résolution quantitative en hydrogène.

Les mesures de réflectométrie optique Brillouin dans le domaine temporel, de réflectométrie optique Brillouin dans le domaine fréquentiel, de réflectométrie optique Brillouin dans le domaine de corrélation, optique Brillouin par analyse dans le domaine temporel, optique Brillouin dans le domaine fréquentiel et optique Brillouin par analyse dans le domaine de corrélation sont plus connues sous leur dénomination anglaise et le sigle correspondant qui sont respectivement, Brillouin Optical Time Domain Reflectometry (BOTDR), Brillouin Optical Frequency Domain Reflectometry (BOFDR), Brillouin Optical Time Domain Analysis (BOTDA), Brillouin Optical Time Frequency Domain Analysis (BOFDA) et Brillouin Optical Correlation Domain (BOCDA). Ces mesures sont des mesures qui sont généralement mises en oeuvre dans les dispositifs de mesure de température à fibre optique et/ou dans les dispositifs de surveillance de déformation.

Le dispositif peut être adapté pour effectuer une mesure de référence d'au moins un paramètre de la fibre optique de mesure à au moins un emplacement de la première fibre optique de mesure, ladite mesure de référence n'étant pas influencée par la présence d'hydrogène.

Dans cette configuration, la mesure d'un paramètre de la première fibre optique de mesure, permet, pour un paramètre influençant la mesure du type Brillouin, de corriger la mesure effectuée selon le principe de la mesure du type Brillouin et ainsi d'améliorer la détection et/ou le dosage de l'hydrogène en limitant, ou en supprimant, l'influence du paramètre mesuré lors de la mesure de référence.

Les paramètres influençant la mesure du type Brillouin peuvent notamment être la température, la déformation de la première fibre optique de mesure et la radioactivité, la pression, l'humidité relative, la teneur en eau de l'atmosphère.

Le système optique peut être adapté pour effectuer la mesure de référence le long de la première fibre optique à deux ou plusieurs longueurs d'onde différentes. Pour ce faire, le dispositif pourrait contenir plusieurs lasers de pompe ou bien stimuler en cascade les raies Brillouin générées.

Une mesure d'un paramètre à deux longueurs d'onde de pompe différentes pour lesquelles la présence de l'hydrogène présente deux influences différentes permet, lorsque cette influence est connue, de supprimer cette influence et de fournir une mesure de référence dudit paramètre qui ne soit pas sensible à l'hydrogène.

Le système optique peut être adapté par ajout d'autres types de fibres optiques placées à proximité de la fibre de mesure de l'hydrogène, pour mesurer la variation d'au moins un autre paramètre d'influence de la fibre optique de mesure, de manière à fournir une mesure de référence de la température et/ou de la déformation et/ou des autres paramètres listés ci-avant le long de la fibre optique de mesure.

Le dispositif peut être adapté pour effectuer une mesure de référence d'au moins un paramètre le long de la première fibre optique selon d'autres mesures de rétrodiffusion, que ce soit le principe de la diffusion Raman ou Rayleigh.

La première fibre optique de mesure peut comporter au moins une portion rendue insensible à l'hydrogène de manière à ce que la mesure d'un paramètre de la première fibre optique de mesure par le système optique au niveau de la dite portion fournisse une mesure de référence.

On entend ci-dessus et dans le reste de ce document, par portion de fibre optique, ou fibre optique, rendue insensible à hydrogène que la portion de fibre optique, ou la fibre optique, présente une configuration telle que la présence d'hydrogène dans l'environnement atmosphérique de ladite portion de fibre optique, ou de ladite fibre optique, n'influence pas la mesure réalisée sur ladite portion de fibre optique ou sur ladite fibre optique.

Une telle portion permet de fournir une mesure de référence sans influence de l'hydrogène pour corriger la mesure du paramètre de la première fibre optique de mesure selon le principe de la mesure du type Brillouin.

Il peut être en outre prévu une fibre optique de référence qui, étant destinée à équiper l'installation, est reliée au système optique, ladite fibre optique de référence étant destinée à la fourniture d'une mesure de référence

Il peut être en outre prévu une fibre optique de référence qui, étant destinée à équiper l'installation, est reliée au système optique, ladite fibre optique de référence étant destinée à la fourniture d'une mesure de référence de la température et/ou de la déformation le long de la première fibre optique de mesure.

Une telle fibre optique de référence en fournissant une mesure d'un paramètre pouvant interférer avec la mesure détection et/ou de dosage d'hydrogène selon le principe de la mesure du type Brillouin, permet de corriger ladite mesure de détection et/ou de dosage le long de la première fibre optique de mesure. Une telle correction permet de limiter, ou supprimer, l'influence du paramètre mesuré lors de la mesure de référence faite sur la fibre optique de référence.

La fibre optique de référence peut être configurée pour présenter une sensibilité réduite à l'hydrogène, préférentiellement pour être rendue insensible à l'hydrogène.

Une telle sensibilité réduite, ou insensibilité, à l'hydrogène de la fibre optique de référence permet de réduire, voire annuler, l'influence de l'hydrogène sur la mesure de référence obtenue au moyen de la fibre optique de référence.

La fibre optique de référence peut comporter un coeur et une gaine dont le matériau est configuré de manière à ce que la fibre optique de référence présente une sensibilité réduite à l'hydrogène préférentiellement nulle.

Selon cette possibilité le coeur et/ou la gaine peut être un verre à base de silice ou de chalcogénure ou de vides remplis d'air ou de liquides (« fibres à trous »).

La fibre optique de référence peut comporter un coeur et une gaine dont la répartition en éléments dopants est configurée de manière à ce que la fibre optique de référence présente une sensibilité réduite à l'hydrogène préférentiellement nulle.

La fibre optique de référence peut comporter un revêtement adapté pour limiter la diffusion de l'hydrogène dans la fibre optique de référence.

Il peut en outre être prévu une deuxième fibre optique de mesure qui, étant destinée à équiper l'installation, est reliée au système optique, ladite deuxième fibre optique de mesure étant configurée pour présenter une interaction avec l'hydrogène différente de celle de la première fibre optique de mesure.

Ainsi, avec une telle deuxième fibre optique de mesure, le dispositif donne accès à deux mesures pour lesquelles l'interaction avec l'hydrogène est différente et permet, en fonction de ladite différence d'interaction, de fournir une information complémentaire sur la source d'hydrogène détectée, comme une information temporelle ou une sensibilité optimisée sur une gamme plus étendue qu'avec un dispositif présentant une seule fibre optique de mesure.

La deuxième fibre optique de mesure peut comporter un moyen de limitation de la diffusion d'hydrogène dans ladite deuxième fibre optique de mesure.

Un tel moyen de limitation de la diffusion permet de réduire la vitesse à laquelle va pénétrer l'hydrogène dans la fibre optique fournissant ainsi une information temporelle sur la source d'hydrogène à l'origine de l'hydrogène détecté.

Le moyen de limitation de la diffusion d'hydrogène de la deuxième fibre optique de mesure peut comporter un revêtement de ladite deuxième fibre optique de mesure, ledit revêtement présentant une perméabilité partielle à l'hydrogène.

On entend par perméabilité partielle à l'hydrogène que le revêtement est adapté pour limiter la pénétration de l'hydrogène dans la fibre optique qui est équipée par le dit revêtement, ceci sans pour autant totalement en supprimer la diffusion dans ladite fibre optique.

Un tel revêtement permet de fournir un moyen de limitation de la diffusion d'hydrogène dans la deuxième fibre optique de mesure ceci avec une influence des propriétés optiques de la deuxième fibre optique de mesure qui est faible, voir nulle.

Le moyen de limitation de la diffusion d'hydrogène de la deuxième fibre optique de mesure peut être le choix de fibres optiques dont la structure interne est modifiée, soit par le choix de ses dopants, soit par le choix de sa matrice vitreuse, soit par des traitements préalables, tels qu'une charge en hydrogène préalable qui est configurée pour augmenter la sensibilité à l'hydrogène de ladite deuxième fibre optique de mesure, ou tels qu'une charge en rayonnements ionisants, par exemple des radiations gamma et/ou UV.

Ces traitements préalables permettent de changer le seuil de détection de l'hydrogène et ainsi de changer la sensibilité de la détection et/ou le dosage obtenus à partir des mesures réalisées avec une telle deuxième fibre optique de mesure.

Ces traitements préalables et choix de fibres optiques spécifiques (en nature et en revêtements primaire et secondaire) permettent de changer le seuil de saturation de l'absorption de l'hydrogène.

La deuxième fibre optique de mesure peut présenter une charge en hydrogène préalable configurée pour modifier l'interaction de ladite fibre optique avec l'hydrogène.

La ou les fibres optiques de mesure et la fibre optique de référence peuvent être placées sous la forme d'un faisceau ou d'une nappe.

Un tel placement des fibres optiques entre-elles permet d'offrir un environnement identique pour l'ensemble des fibres optiques du point vu thermique, des contraintes, de la pression et du niveau de radiation auxquelles elles sont soumises. Ainsi, pour un dispositif comportant une fibre optique de référence, la mesure de référence est réalisée dans des conditions identiques à celles utilisées pour la détection et/ou le dosage d'hydrogène. De même pour un dispositif comportant une deuxième fibre optique de mesure, les mesures réalisées par la première et la deuxième fibre optique de mesure sont comparables entre-elles.

L'invention concerne également un procédé de détection et/ou dosage d'hydrogène, pour surveiller une installation, ledit procédé comportant les étapes consistant à :

- installer une première fibre optique de mesure dans l'installation,
- réaliser une mesure d'un paramètre le long de la fibre optique de mesure selon le principe de la mesure du type Brillouin,

L'invention concerne également un procédé de détection et/ou de dosage d'hydrogène mettant en oeuvre un dispositif selon l'invention et comportant les étapes consistant à :

- mettre en oeuvre le système optique pour mesurer un paramètre de la première fibre optique de mesure le long de la première fibre optique de mesure selon le principe de la mesure Brillouin,
- respectivement détecter et/ou doser l'hydrogène dans la fibre optique de mesure à partir de du paramètre mesuré le long de la fibre optique de mesure selon le principe de la mesure du type Brillouin.

Un tel procédé permet de réaliser une détection et/ou un dosage d'hydrogène dans une installation quelle que soit l'étendue de la zone à surveiller de l'installation. Il permet également d'identifier, lorsqu'il y a détection de la présence d'hydrogène, l'endroit, le long de la première fibre optique de mesure, auquel a eu lieu la détection d'hydrogène.

Il peut être mis en oeuvre un dispositif adapté pour effectuer une mesure de référence, l'étape respective de détection et/ou dosage l'hydrogène comportant les sous-étapes consistant à :

- mettre en oeuvre le système optique pour effectuer une mesure de référence le long de la première fibre optique de mesure,
- corriger sur la base de la mesure de référence la mesure du paramètre de la première fibre optique de mesure effectuée le long de la première fibre optique de mesure selon le principe de la mesure du type Brillouin,
- respectivement détecter et/ou mesurer la présence d'hydrogène dans la première fibre optique de mesure à partir de la mesure du paramètre de la première fibre optique de mesure le long de la première fibre optique de mesure selon le principe de la mesure du type Brillouin qui a été corrigée à partir de la mesure de référence.

Un procédé comportant une telle étape de correction de la mesure de variation d'indice permet de limiter les risques de détection erronée d'une présence d'hydrogène qui serait liée, par exemple, à un changement de température.

**BRÈVE DESCRIPTION DES DESSINS**

La présente invention sera mieux comprise à la lecture de la description d'exemples de réalisation, donnés à titre purement indicatif et nullement limitatif, en faisant référence aux dessins annexés sur lesquels :

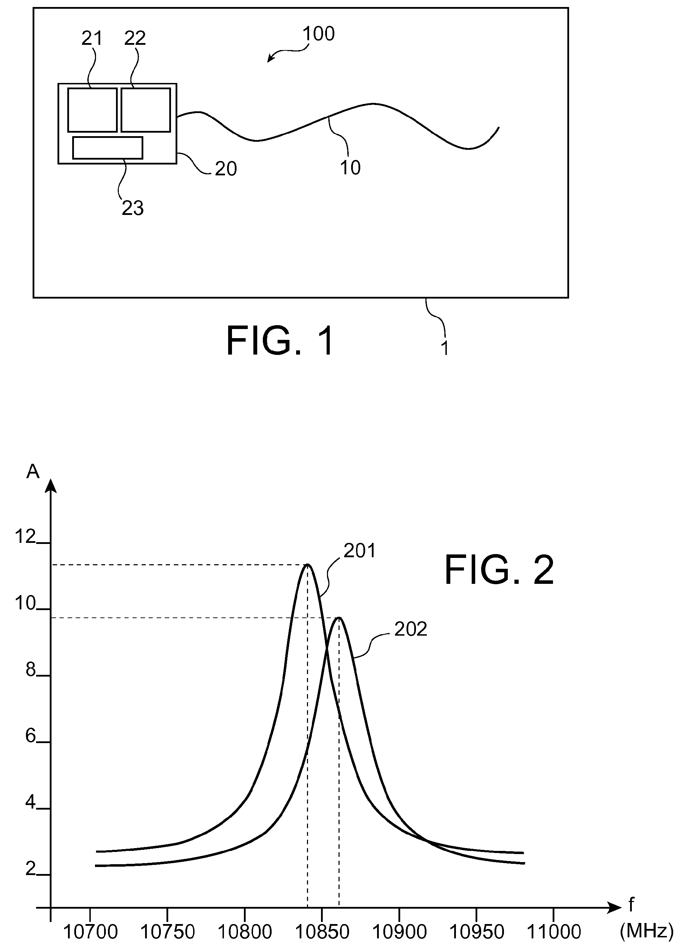

- la figure 1 illustre schématiquement un dispositif selon l'invention dans lequel il est prévu une seule fibre optique de mesure, ledit système comportant un système optique adapté pour effectuer une mesure de réflectométrie selon le principe Brillouin,
- la figure 2 illustre deux spectres d'un même pic de rétrodiffusion Brillouin obtenus respectivement par la mise en oeuvre d'un dispositif tel qu'illustré sur la figure 1 lors de respectivement la mise en présence de la première fibre optique de mesure avec une forte quantité d'hydrogène et après dégazage de l'hydrogène de ladite première fibre optique de mesure,

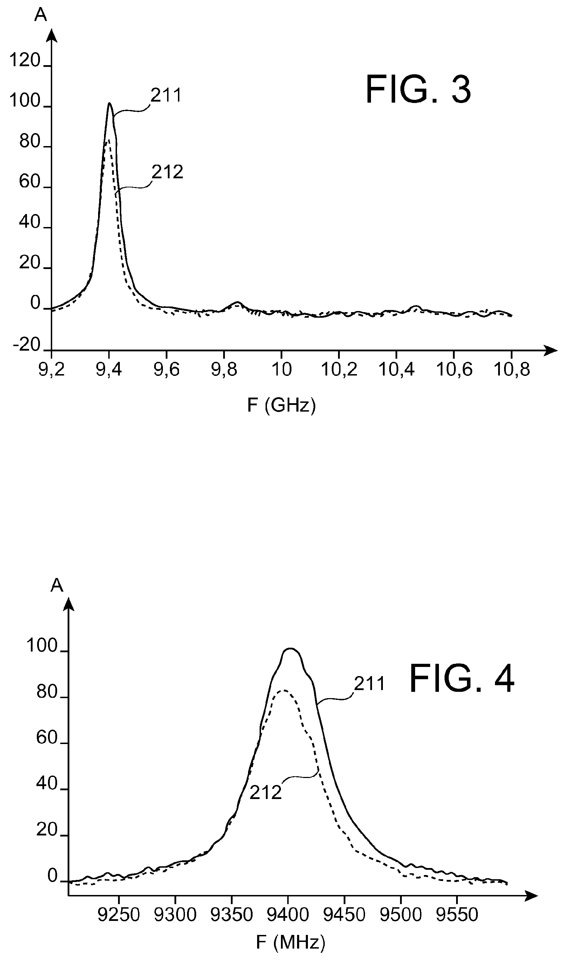

- la figure 3 illustre deux spectres de mesure du type Brillouin présentant tous deux deux mêmes pics Brillouin, ces deux spectres ayant été respectivement obtenus avec un dispositif tel qu'illustré sur la figure 1 ceci respectivement avant et pendant une exposition de la première fibre optique de mesure du dispositif à une atmosphère contenant 32% d'hydrogène,
- la figure 4 illustre une vue rapprochée centrée sur le pic Brillouin principal des spectres présents sur la figure 3,

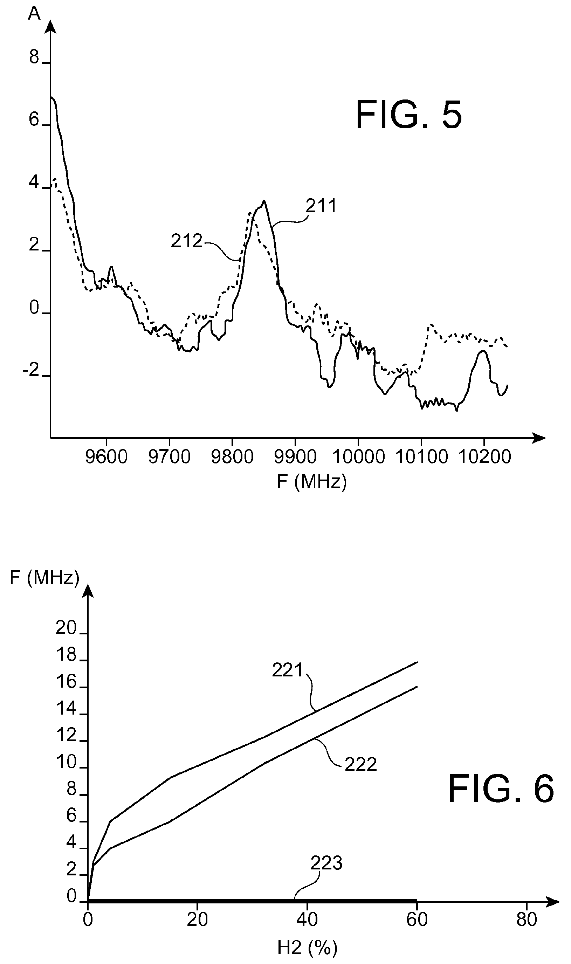

- la figure 5 illustre une vue rapprochée centrée sur le pic Brillouin secondaire des spectres présents sur la figure 3,
- la figure 6 illustre la variation de la fréquence du pic principal avec la proportion en hydrogène atmosphérique lors de la mesure du type Brillouin avec un dispositif tel qu'illustré sur la figure 1 ceci pour deux types de première fibre optique de mesure et pour une fibre optique rendue insensible à l'hydrogène,

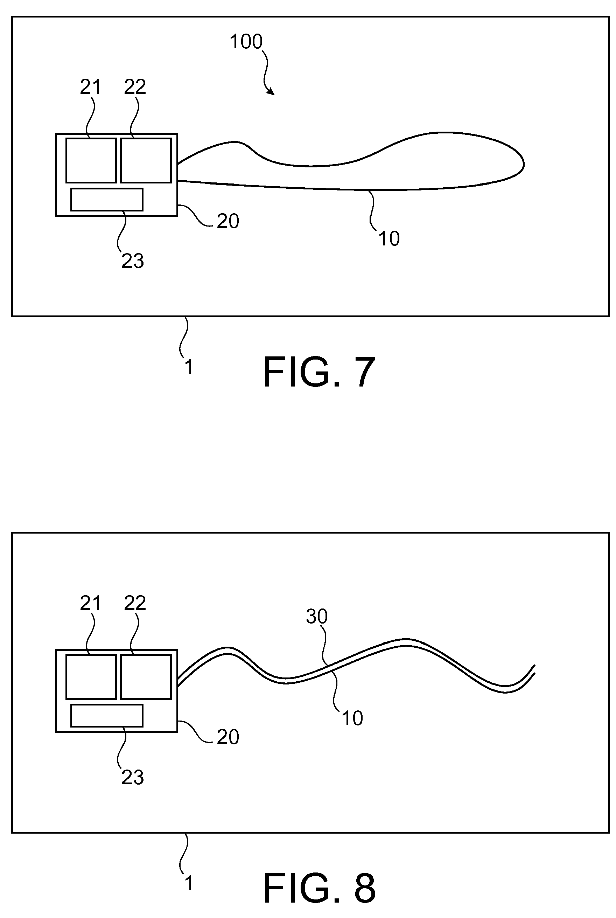

- la figure 7 illustre un dispositif selon l'invention dans lequel il est prévu une seule fibre optique de mesure, ledit système comportant un système optique adapté pour effectuer une mesure optique d'analyse selon le principe Brillouin,
- la figure 8 illustre un dispositif selon un deuxième mode de réalisation de l'invention dans lequel le dispositif comporte une fibre optique de mesure et une fibre optique de référence, le système optique étant adapté pour effectuer une mesure de réflectométrie selon le principe de la mesure du type Brillouin,

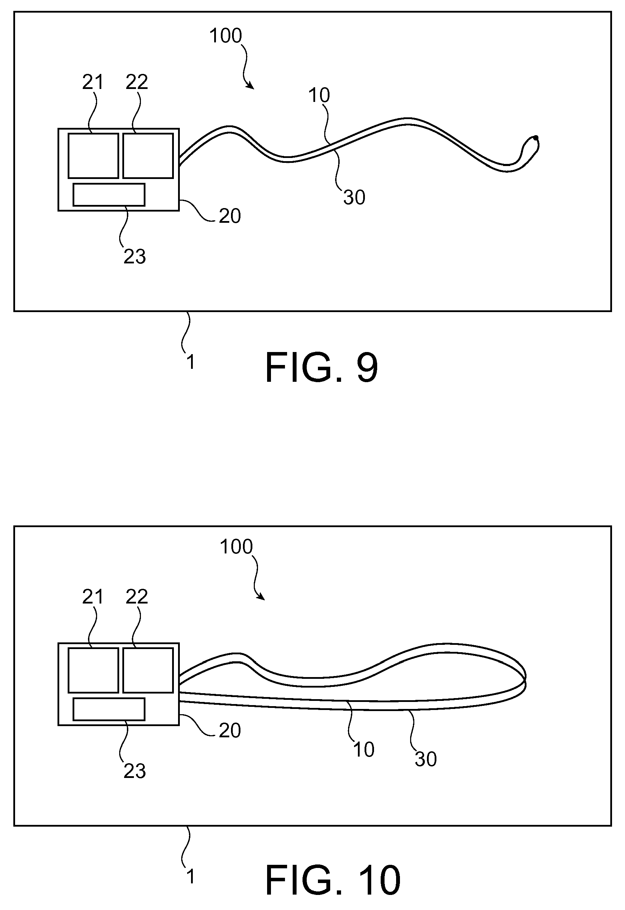

- la figure 9 illustre un dispositif selon un deuxième mode de réalisation de l'invention dans lequel le dispositif comporte une fibre optique de mesure et une fibre optique de référence reliées l'une à l'autre, le système optique étant adapté pour effectuer une mesure d'analyse selon le principe de la mesure du type Brillouin le long de la boucle formée par la fibre optique de mesure et la fibre optique de référence,
- la figure 10 illustre un dispositif selon un deuxième mode de réalisation de l'invention dans lequel le dispositif comporte une fibre optique de mesure et une fibre optique de référence reliées chacune indépendamment au système optique, le système optique étant adapté pour effectuer une mesure d'analyse selon le principe de la mesure du type Brillouin le long de la fibre optique de mesure et de la fibre optique de référence,

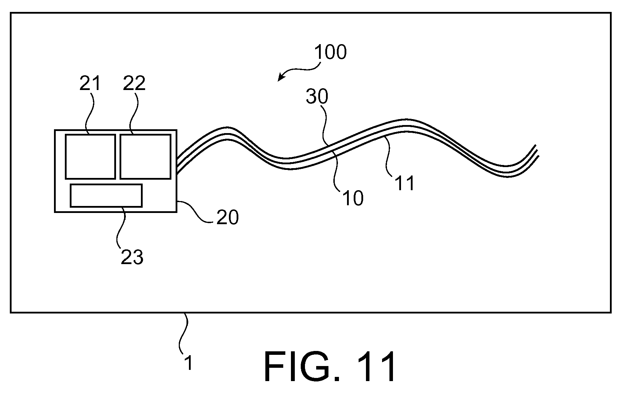

- la figure 11 illustre un dispositif selon un troisième mode de rélisation de l'invention dans lequel le dispositif comporte une première et une deuxième fibre optique de mesure et une fibre optique de référence, le système optique étant adapté pour effectuer une mesure de réflectométrie selon le principe de la mesure du type Brillouin le long des première et deuxième fibres optiques de mesure et de la fibre optique de référence.

Des parties identiques, similaires ou équivalentes des différentes figures portent les mêmes références numériques de façon à faciliter le passage d'une figure à l'autre.

Les différentes parties représentées sur les figures ne le sont pas nécessairement selon une échelle uniforme, pour rendre les figures plus lisibles.

Les différentes possibilités (variantes et modes de réalisation) doivent être comprises comme n'étant pas exclusives les unes des autres et peuvent se combiner entre elles.

## EXPOSÉ DÉTAILLÉ DE MODES DE RÉALISATION PARTICULIERS

La figure 1 illustre un dispositif 100 de détection et/ou de dosage d'hydrogène équipant une zone à surveiller d'une installation 1, telle qu'un lieu de stockage, un forage géothermique, un lieu d'entreposage ou un réservoir industriel de produits chimiques et/ou radioactifs. Le dispositif 100 illustré sur la figure 1 est un dispositif selon un premier mode de réalisation de l'invention. La figure 1 illustre une première possibilité de ce premier mode de réalisation dans laquelle la configuration du dispositif est adaptée pour effectuer une mesure du type Brillouin selon le principe de la réflectométrie.

Un tel dispositif 100 comporte :

- une première fibre optique de mesure 10 équipant l'installation 1,
- un système optique 20 relié à la première fibre optique de mesure 10 et adapté pour effectuer une mesure du type Brillouin.

On peut noter que dans le dispositif selon la configuration illustrée sur la figure 1, le système optique est adapté pour une mesure en configuration de réflectométrie, la fibre optique n'étant reliée au système que par une seule de ses extrémités.

La première fibre optique de mesure 10 est une fibre optique adapté à la longueur d'onde de travail du système optique 20. La fibre optique peut être, par exemple similaire à celles généralement utilisées pour effectuer des mesures de température de type Brillouin.

Pour des installations présentant une accessibilité réduite, la première fibre optique est préférentiellement adaptée pour autoriser une mesure durable et stable pouvant dépasser plusieurs dizaines d'années ceci sans nécessiter une quelconque maintenance.

La première fibre optique de mesure 10 comporte classiquement un coeur, une gaine optique et un revêtement de protection. Le coeur et la gaine peuvent être en dioxyde de silicium, le coeur présentant un indice de réfraction plus important que celui de la gaine de manière à confiner la lumière dans le coeur. Les fibres optiques monomodes à gradient d'indice conviennent également. Le changement d'indice de réfraction peut être obtenu par l'incorporation d'éléments dopants classiquement utilisés pour ce type d'application, par exemple au germanium, au phosphore, au fluor, à l'aluminium etc. Le revêtement peut être adapté aux conditions d'exploitation pour limiter les endommagements de la première fibre optique de mesure 10 qui pourraient être liés aux conditions dans lesquelles est installée la première fibre optique de mesure 10. Un tel revêtement, pour une installation ne présentant pas de conditions préjudiciables pour la première fibre optique de mesure 10, est par exemple un simple revêtement plastique tel que des acrylates ou polyimides.

Le revêtement de la première fibre optique peut également être un revêtement spécifiquement adapté pour augmenter la sensibilité à l'hydrogène, par exemple un revêtement en palladium. La fibre optique peut aussi être constituée de verres spécifiques, par exemple des verres à base de chalcogénures. Les « fibres à trous » (réalisées en laissant des vides - éventuellement ultérieurement comblées) conviennent également.

La première fibre optique de mesure 10 est adaptée pour guider les rayonnements électromagnétiques aux longueurs d'onde auxquelles émet le système optique 20.

La première fibre optique de mesure 10 peut présenter une adaptation pour que le spectre de rétrodiffusion Brillouin, ou le gain Brillouin, soit facilement mesurable ceci en modifiant la proportion en élément dopant le long de son rayon. Avec une telle adaptation, la première fibre optique de mesure 10 peut notamment présenter les différents modes acoustiques donnant lieu à des raies Brillouin sont spectralement séparés ou bien évoluent de manière sensiblement identique avec la diffusion d'hydrogène dans la fibre optique.

Pour limiter l'influence des contraintes appliquées sur la première fibre optique de mesure 10, la première fibre optique de mesure 10 peut être équipée d'une gaine intermédiaire flottante (également connue sous la dénomination anglaise de « loose tube ») de manière à réduire les contraintes auxquelles est soumise la première fibre optique de mesure 10. On limite ainsi l'influence que de telles contraintes pourraient avoir sur la détection d'hydrogène.

La première fibre optique de mesure 10 est déployée dans l'installation sur une zone à surveiller. Ce déploiement est réalisé de sorte à couvrir le maximum de la surface de la zone à surveiller et ainsi garantir la détection d'une éventuelle fuite ou dégagement d'hydrogène dans ladite zone.

La première fibre optique de mesure 10 comporte une première et une seconde extrémité, la première extrémité étant reliée au système optique 20 pour une mesure du type Brillouin de type réflectométrie dans une configuration telle qu'illustrée sur la figure 1.d

Le système optique 20 est un système optique adapté pour effectuer une mesure du type Brillouin de manière résolue spatialement le long de la première fibre optique de mesure.

Le système optique est adapté en fonction du type de mesure du type Brillouin. Ainsi, dans la configuration illustrée sur la figure 1, le système optique est adapté pour effectuer une mesure de réflectométrie de type Brillouin telle que la mesure de réflectométrie optique Brillouin dans le domaine temporel (sigle anglais BOTDR), la mesure de réflectométrie optique Brillouin dans le domaine fréquentiel (sigle anglais BOFDR) et la mesure de réflectométrie optique Brillouin dans le domaine de corrélation (BOCDR).

A cet effet, le système optique 20 comporte :

- un moyen d'émission 21 de lumière, tel qu'un laser, adapté pour émettre au moins un rayonnement électromagnétique,
- un moyen de mesure 22 optique adapté pour détecter et mesurer un rayonnement électromagnétique, tel qu'un système d'analyse spectrale, un système d'analyse de gain ou un système d'analyse de pertes, et
- un moyen de commande et d'analyse 23, adapté pour commander le moyen d'émission et le moyen de mesure, et pour analyser les mesures effectuées par le moyen de mesure 22.

Le moyen d'émission 21 comporte généralement un laser dont la longueur d'onde peut être fixe ou accordable en fonction des possibilités de l'invention. Le moyen, en fonction du type de mesure Brillouin peut comporter un ou plusieurs lasers qui émettent en continu ou en pulsé. Parmi ce ou ces lasers, un laser est dit principal, également dénommé pompe, et émet un rayonnement principal à une longueur d'onde principale.

Pour les applications visées, la longueur d'onde d'émission principale du moyen d'émission est une longueur d'onde classiquement utilisée pour la mesure de température selon le principe de la mesure de température du type Brillouin. Dans le but d'obtenir une sensibilité maximum à la présence d'hydrogène la longueur d'onde d'émission principale peut être choisie dans une gamme de longueur d'onde à laquelle la diffusion d'hydrogène présente une sensibilité maximale, telle que celles qui sont mentionnées dans la demande internationale WO 2009/067671. Selon ce principe, la longueur d'onde d'émission peut être sensiblement égale à 1,2 $\mu$m.

Le moyen de mesure 22 est adapté pour détecter et/ou mesurer un rayonnement électromagnétique à une longueur d'onde très proche de celle émise par le moyen d'émission, typiquement proche de l'ordre de 10GHz et généralement compris entre 9 et 13 Ghz. Un tel moyen de mesure 22 est adapté au type de la mesure Brillouin. Ainsi, par exemple, pour un système optique adapté pour effectuer une mesure de type BOTDR, le moyen de mesure est un système d'analyse spectrale. Ce type de moyen de mesure 22 étant connu par ailleurs pour les capteurs de température à fibre optique de type Brillouin, il n'est pas décrit plus en détail dans ce document.

Le moyen de commande et d'analyse 23 est adapté pour commander le moyen d'émission 21 et le moyen de mesure 22 de manière à effectuer une mesure d'un paramètre de la première fibre optique de mesure 10 selon le principe de la mesure du type Brillouin. Le moyen de commande et d'analyse 23, avec une telle adaptation est apte à commander le moyen d'émission 21 de manière à émettre un rayonnement électromagnétique adapté et à commander le moyen de mesure 22 de manière à détecter et mesurer le rayonnement électromagnétique issu du phénomène du type Brillouin, qu'il soit de la rétrodiffusion, du gain ou de la perte Brillouin, qui a lieu le long de la première fibre optique de mesure 10 lors du passage du rayonnement électromagnétique.

Une telle adaptation du moyen de commande et d'analyse 23 étant identique à celle mise en oeuvre lors d'une mesure de température par un capteur à fibre optique du type Brillouin, elle n'est pas explicitée plus en détail dans ce document

Le moyen de commande et d'analyse 23 est en outre adapté pour analyser les mesures effectuées par le moyen de détection 22 de manière à identifier une potentielle diffusion d'hydrogène à un endroit de la première fibre optique de mesure pour détecter une variation de l'indice effectif de propagation du mode optique $n_{eff}$ à un endroit de cette même fibre optique 10.

Selon une possibilité de l'invention dans laquelle l'installation présente une température qui peut localement ou globalement varier, une telle variation pouvant entraîner une variation de la mesure du type Brillouin le long de la première fibre optique de mesure 10, le dispositif peut être adapté pour mesurer un paramètre à au moins un emplacement de la première fibre optique de mesure 10 de manière à fournir une mesure de référence, cette mesure de référence étant de préférence une mesure de température et/ou de déformation de la première fibre optique de mesure 10.

Selon cette possibilité, une telle mesure, pour servir de mesure de référence, ne doit pas être influencée par la présence d'hydrogène. Une telle condition peut, par exemple, être obtenue, en mettant en oeuvre une mesure de température le long de la première fibre optique en utilisant une mesure utilisant deux longueurs d'onde d'émission différentes du moyen d'émission 22, ceci au moyen d'une mesure qui peut être autre qu'une mesure du type Brillouin telle que cela est décrit dans le document WO 2011/115686.

Selon cette même possibilité, la première fibre optique de mesure 10 peut présenter des portions régulièrement réparties sur toute sa longueur qui sont rendues insensibles à l'hydrogène et qui servent à fournir la mesure de référence pour corriger la mesure le long des portions de la première fibre de mesure 10 qui ne sont pas rendues insensibles à l'hydrogène. Les portions qui sont rendues insensibles peuvent l'être soit par une modification de la configuration de la fibre optique en elle-même, selon le principe décrit dans le document EP 1195628, ou par la présence d'un revêtement adapté sur la surface desdites portions, tel que celui décrit dans le document EP 1426804.

La figure 2 illustre un exemple de mesures faites lors de mise en oeuvre d'un dispositif 100 selon ce premier mode de réalisation. L'axe des ordonnées représente l'amplitude A de l'intensité du rayonnement électromagnétique rétrodiffusé en unité arbitraire et l'axe des abscisses représente la fréquence f de décalage en MHz du rayonnement rétrodiffusé par rapport au rayonnement émis par le moyen d'émission 21. Lors de cette mise en oeuvre du dispositif 100, la première fibre optique de mesure 10 a été mise en présence à une forte pression d'hydrogène (150 bar) pendant une durée de 7 jours de manière à saturer la fibre optique en hydrogène. Puis, la première fibre optique de mesure 10 a été remise à l'air durant cette même durée de manière à ce que l'hydrogène dégaze hors de la première fibre optique de mesure 10. Le premier spectre 202 et le second spectre 201, illustrés sur cette figure, ont été réalisés respectivement à l'issue des 7 jours d'exposition et à l'issue de la durée de remise à l'air.

Ainsi, le premier spectre 202 montre un décalage en fréquence du pic de rétrodiffusion Brillouin correspondant à une saturation en hydrogène de la première fibre optique de mesure 10 tandis que le second spectre 201 correspond au pic de rétrodiffusion Brillouin observé en l'absence d'hydrogène dans ladite première fibre optique de mesure 10.

On peut ainsi voir sur cette figure que, lors de la mise en présence de la première fibre optique de mesure 10 avec l'hydrogène, la diffusion d'hydrogène entraîne une augmentation de la fréquence Brillouin et une diminution de l'intensité de ce même pic. Cette double influence est directement liée à l'évolution de l'indice effectif de propagation du mode optique $n_{eff}$ qui intervient dans le décalage en fréquence Brillouin selon la formule

suivante $\nu_B = \dfrac{2n_{eff}V_a}{\lambda}$ avec $\nu_B$ la fréquence de décalage Brillouin, $V_a$ la vitesse des ondes acoustiques, c'est-à-dire des phonons à l'origine du phénomène de rétrodiffusion Brillouin, et $\lambda$ la longueur d'onde du rayonnement électromagnétique émis.

Les mesures présentes sur la figure 2 permettent d'illustrer les différentes possibilités d'adaptation du moyen de contrôle et d'analyse afin de détecter et/ou d'évaluer une variation de l'indice effectif de propagation de l'onde optique $n_{eff}$. En effet, la détection et/ou l'évaluation de la variation de l'indice de réfraction peuvent être obtenues soit :

- a) par une mesure directe de la variation de la fréquence Brillouin du pic principal ou des pics secondaires,
- b) par une mesure de l'intensité du pic Brillouin du pic principal ou de l'un des pics secondaires,
- c) par une mesure d'intensité à une fréquence prédéterminée correspondant sensiblement à celle du pic Brillouin principal ou à l'un des pics secondaires en l'absence d'hydrogène, selon cette dernière possibilité la chute d'intensité sera liée à la fois au décalage en fréquence et à la baisse d'intensité du pic Brillouin.

La figure 3 et les vue rapprochées de cette dernière qui sont présentes sur les figures 4 et 5 illustrent la possibilité de fonctionner aussi bien sur le pic Brillouin principal que sur le pic Brillouin secondaire. Les figures 4 et 5 sont des vues rapprochées de la figure 3 centrées sur respectivement la fréquence des pics Brillouin principal et secondaire.

Sur les figures 3 à 5, l'axe des ordonnées représente l'amplitude A de l'intensité du rayonnement électromagnétique rétrodiffusé en unité arbitraire et l'axe des abscisses représente la fréquence f, ceci en GHz pour la figure 3 et en MHz pour les figures 4 et 5, du rayonnement rétrodiffusé par rapport au rayonnement émis par le moyen d'émission 21.

Les mesures qui figurent sur les figures 3 à 5 sont deux mesures 211 et 212 de type Brillouin obtenues lors la mise en présence de la première fibre optique de mesure 10 avec une atmosphère contenant respectivement 32% et 0% d'hydrogène. On peut ainsi voir que lors de cette mise en présence de la première fibre optique de mesure 10 avec l'hydrogène que la diffusion d'hydrogène entraîne un décalage et une variation d'intensité à la fois sur le pic Brillouin principal et secondaire. Ainsi, le système de mesure 22 peut être adapté pour réaliser une mesure du type Brillouin soit sur le pic principal, soit sur l'un des pics secondaires, soit à la fois sur le pic principal et sur l'un, ou plusieurs, des pics secondaires.

Ainsi, après équipement de l'installation par le dispositif, la détection et/ou le dosage en hydrogène sont réalisés selon un procédé comportant les étapes consistant à :

- mettre en oeuvre le système optique pour mesurer une variation d'un paramètre de la première fibre optique de mesure 10 le long de la première fibre optique de mesure 10 selon le principe de la mesure du type Brillouin,
- détecter et/ou doser l'hydrogène dans la première fibre optique de mesure 10 à partir de la mesure selon le principe de la mesure du type Brillouin le long de la première fibre optique de mesure 10.

Pour un système optique adapté pour effectuer une mesure de référence, l'étape consistant à mesurer et/ou détecter la présence d'hydrogène dans la première fibre optique de mesure 10 comporte les sous étapes consistant à :

- mettre en oeuvre le système optique pour effectuer la mesure de référence le long de la première fibre optique de mesure 10,
- corriger la mesure de variation du paramètre de la fibre optique de mesure 10 le long de la première fibre optique de mesure 10 sur la base de la mesure de référence,
- détecter et/ou doser l'hydrogène dans la première fibre optique de mesure 10 à partir de la mesure du paramètre selon le principe de la mesure du type Brillouin corrigée le long de la première fibre optique de mesure 10.

Dans l'étape de mise en oeuvre du système optique pour effectuer la mesure de référence, ladite mesure peut être réalisée simultanément à la mesure de variation de l'indice effectif de propagation du mode optique $n_{eff}$ le long de la première fibre optique de mesure 10 si la mesure de référence est obtenue au moyen de portions de la première fibre optique de mesure 10 rendues insensibles à l'hydrogène.

Il est à noter que pour une mesure précise de la quantité d'hydrogène absorbée à un emplacement de la fibre optique, il est également nécessaire de prévoir une étape préalable d'étalonnage du dispositif 100.

La figure 6 est un exemple illustrant une telle étape pour trois fibres optiques différentes, une première comportant un dopage à base de fluor, une deuxième comportant un dopage à base de germanium et une troisième, comme nous le verrons par la suite qui a été rendue insensible à l'hydrogène.

Sur la figure 6, l'axe des ordonnées représente la fréquence f de décalage en MHz du rayonnement rétrodiffusé par rapport au rayonnement émis par le moyen d'émission 21 et l'axe des abscisses représente la quantité d'hydrogène à laquelle la fibre optique a été soumise.

Ainsi, on peut voir sur la figure 6, que les deux fibres optiques, si elles présentent une variation du décalage en fréquence du pic principal Brillouin d'apparence générale similaire, ces deux fibres ne possèdent pas une interaction identique avec l'hydrogène démontrant, pour l'obtention d'une mesure précise de la quantité d'hydrogène avec un dispositif selon l'invention, la nécessité d'une telle étape d'étalonnage. De plus, les mesures présentes sur la figure 6 montrent également la non linéarité de la variation d'un paramètre mesuré selon une mesure du type Brillouin avec la quantité d'hydrogène à laquelle est soumise la fibre optique.

Une telle étape d'étalonnage étant accessible à l'homme du métier, elle n'est pas décrite de façon plus détaillé dans ce document.

La figure 7 illustre un dispositif 100 selon une deuxième possibilité du premier mode de réalisation dans laquelle la configuration du dispositif 100 est adaptée pour effectuer une mesure du type Brillouin qui est réalisée par analyse dans le domaine temporel, fréquentiel ou de la corrélation.

Une telle configuration est adaptée pour un dispositif effectuant la mesure du paramètre selon le principe de mesure du type Brillouin qui est une mesure optique Brillouin par analyse dans le domaine temporel (BOTDA), une mesure optique Brillouin dans le domaine fréquentiel (BOFDA) ou une mesure optique Brillouin par analyse dans le domaine de la corrélation (BOCDA).

Un dispositif selon cette deuxième possibilité du premier mode réalisation se différencie d'un système selon la première possibilité en ce que la première fibre optique de mesure 10 est reliée au système optique par ses deux extrémités pour permettre l'analyse du rayonnement électromagnétique sortant de la fibre optique. Le système optique 20 présente une configuration similaire à celle d'un système optique d'un capteur de température à fibre optique effectuant la mesure du type Brillouin par analyse dans le domaine correspondant (c'est-à-dire temporel, fréquentiel, ou de la corrélation).

De même que pour un dispositif présentant une configuration selon la première possibilité, une portion de la première fibre optique peut être rendue insensible à l'hydrogène. Une telle portion est avantageusement la deuxième moitié de la première fibre optique de mesure. Ainsi il est possible, en installant la deuxième partie de la première fibre optique de mesure 10 le long de sa première partie, de réaliser la mesure de référence sur toute la longueur de la première partie de la première fibre optique de mesure 10.

La figure 8 illustre un dispositif selon un deuxième mode de réalisation dans lequel il est prévu une fibre optique de référence 30. La figure 8 illustre une première possibilité du deuxième mode de réalisation dans laquelle la configuration du dispositif est adaptée pour effectuer une mesure du type Brillouin selon le principe de la réflectométrie.

Un dispositif selon ce deuxième mode de réalisation se différencie d'un dispositif selon le premier mode de réalisation en ce qu'il comporte une fibre optique de référence 30 reliée au système optique et en ce que le système optique 20 est adapté pour effectuer une mesure de référence le long de la fibre optique de référence 30.

Selon la première possibilité de ce deuxième mode de réalisation, la fibre optique de référence 30 est une fibre optique configurée pour présenter une sensibilité réduite à l'hydrogène. Une telle configuration de la fibre optique de référence 30 peut être obtenue soit par une configuration modifiée de la fibre en elle-même, selon le principe décrit dans le document EP 1195628, ou par la présence d'un revêtement adapté sur la surface de la fibre optique de référence, tel que celui décrit dans le document EP 1426804.

La fibre optique de référence 30 présente préférentiellement, pour faciliter la correction de la mesure de variation d'indice effectif de propagation du mode optique $n_{eff}$ le long de la première fibre optique de mesure 10, des caractéristiques similaires à celles de la première fibre optique de mesure 10.

La première fibre optique de mesure 10 et la fibre optique de référence 30 sont, lors de l'équipement de l'installation par le dispositif, installées côte à côte. Pour facilité un tel équipement de l'installation la première fibre optique de mesure 10 et la fibre optique de référence 30 peuvent être placées sous la forme d'un faisceau de fibres ou sous la forme d'une nappe.

La fibre optique de référence 30 est reliée par une extrémité au système optique.

Le système optique 20 est préférentiellement adapté pour effectuer une mesure, dite de référence, de la variation du paramètre selon la mesure du type Brillouin le long de la fibre optique de référence 30 dans des conditions identiques à la mesure du paramètre selon la mesure du type Brillouin le long de la première fibre optique de mesure 10. Il est également possible, sans que l'on sorte du cadre de l'invention, que le système optique soit adapté pour mesurer un paramètre le long de la fibre optique de référence 30, tel que la température, de manière à corriger les variations du paramètre mesuré le long de la première fibre optique de mesure 10 selon le principe de la mesure du type Brillouin qui sont uniquement liées à ce paramètre mesuré le long de la fibre optique de référence 30.

Selon la possibilité de l'invention dans laquelle la première fibre optique de référence est équipée d'une gaine intermédiaire flottante, la fibre optique de référence peut également être équipée d'un tel équipement.

La mise en oeuvre d'un dispositif selon ce deuxième mode de réalisation est similaire à celui d'un dispositif selon le premier mode de réalisation qui comporte les étapes de mise en oeuvre du système optique 20 pour effectuer la mesure de référence et de correction du paramètre le long de la première fibre optique de mesure 10.

Les figures 9 et 10 illustrent respectivement une deuxième et une troisième possibilité du deuxième mode de réalisation dans lesquelles la configuration du dispositif 100 est adaptée pour effectuer une mesure du type Brillouin qui est réalisée par analyse dans le domaine temporel, fréquentiel ou de la corrélation.

De telles deuxième et troisième possibilités du deuxième mode de réalisation se différencient de la première possibilité d'un système selon le deuxième mode de réalisation en ce que la première fibre optique de mesure 10 est reliée au système optique 20 par ses deux extrémités pour permettre l'analyse du rayonnement sortant de la fibre optique. Le système optique 20 présente une configuration similaire à celle d'un système optique d'un capteur de température à fibre optique effectuant la mesure du type Brillouin par analyse dans le domaine correspondant (c'est-à-dire temporel, fréquentiel, ou de la corrélation).

La figure 9 illustre la possibilité selon laquelle la première fibre optique de mesure 10 est couplée optiquement, par son extrémité qui n'est pas reliée au système optique 20, à une extrémité de la fibre optique de référence 30. L'extrémité de la fibre optique de référence qui n'est pas reliée à la première fibre optique de mesure 10 est reliée au système optique 20. Ainsi on se retrouve dans une configuration similaire à celle de la deuxième possibilité du premier mode de réalisation pour laquelle la deuxième partie de la fibre optique à été rendue insensible à l'hydrogène.

La figure 10 illustre la troisième possibilité du deuxième mode de réalisation pour laquelle la première fibre optique de mesure 10 et la fibre optique de référence 30 sont reliées indépendamment l'une de l'autre au système optique 10. Le système optique, de même que pour la première possibilité du deuxième mode de réalisation, est adapté pour effectuer indépendamment la mesure du paramètre le long de la première fibre optique de mesure 10 et la mesure de référence au moyen de la fibre optique de référence 20.

La figure 11 illustre un dispositif selon un troisième mode de réalisation dans lequel le dispositif comporte une deuxième fibre optique de mesure 11.

Le dispositif 100, tel qu'illustré sur la figure 11, et de manière similaire aux premières possibilités du premier et du deuxième mode de réalisation, présente une configuration adaptée pour effectuer une mesure du type Brillouin selon le principe de la réflectométrie.

Un dispositif selon le troisième mode de réalisation se différencie d'un dispositif selon le deuxième mode de réalisation en ce qu'il comporte une deuxième fibre optique de mesure 11 présentant une interaction à l'hydrogène différente de celle de la première fibre optique de mesure 10. Le système optique est en outre adapté pour effectuer une mesure d'un paramètre selon la mesure du type Brillouin le long de la deuxième fibre optique de mesure 11, ce paramètre étant identique à celui mesuré le long de la première fibre optique de mesure 10.

La deuxième fibre optique de mesure 11 présente une interaction différente à l'hydrogène qui peut être de différents types.

Un premier type d'interaction différente de la première fibre optique de mesure 10 est une diffusion de l'hydrogène réduite dans la deuxième fibre optique de mesure 11. Un tel type d'interaction peut être obtenu au moyen d'une barrière de diffusion tel qu'un revêtement de la deuxième fibre optique de mesure 11 adéquat présentant une perméabilité partielle à l'hydrogène. Un tel revêtement peut être un revêtement de carbone dont les caractéristiques, telles que l'épaisseur et la densité, sont choisies pour fournir une réduction de la diffusion de l'hydrogène dans la deuxième fibre optique de mesure 11 sans totalement la supprimer. On peut voir l'effet d'un tel revêtement sur la figure 6 qui montre que le décalage en fréquence 223 du pic Brillouin pour une fibre optique rendue insensible à l'hydrogène au moyen d'un tel revêtement est constant et n'est pas impacté par la présence d'hydrogène.

Le deuxième type d'interaction différente de la première fibre optique de mesure 10 est une sensibilité réduite de la deuxième fibre optique de mesure 11 à l'hydrogène. Une telle sensibilité réduite de la deuxième fibre optique de mesure 11 peut être obtenue avec une configuration de la deuxième fibre optique de mesure 11 qui est différente de celle de la première fibre optique de mesure 10. Une telle différence de configuration est par exemple une composition modifiée du coeur et/ou de la gaine de la fibre optique telle que le décrit le document EP 1195628.

Bien entendu, et contrairement à ce qui est décrit dans EP 1195628, les compositions de ces différentes parties de la deuxième fibre optique de mesure 11 sont adaptées pour que ladite fibre optique 11 présente une sensibilité à l'hydrogène non nulle. On peut également noter que selon ce même principe il est également envisageable d'augmenter la sensibilité à l'hydrogène de la deuxième fibre optique de mesure 11 par l'utilisation d'une configuration de cette dernière qui est différente.

Un troisième type d'interaction différente est la variation d'indice effectif de propagation du mode optique $n_{eff}$ pour une même quantité d'hydrogène, obtenue par exemple par compactage du matériau formant le coeur et la gaine de la fibre optique (par exemple la silice). Ce troisième type d'interaction différente peut également être obtenu par un pré-chargement de la deuxième fibre optique de mesure 11 par de l'hydrogène ou du deutérium, ce pré-chargement étant rendu définitif par, par exemple, un traitement à un rayonnement ultraviolet.

Ainsi, pour une même quantité d'hydrogène absorbée par la première et la deuxième fibre optique de mesure 11, la deuxième fibre optique de mesure 11, présente une variation d'indice effectif du mode de propagation optique qui est équivalente à celle de la pré-charge en hydrogène à laquelle s'ajoute la quantité d'hydrogène absorbée. La variation d'indice effectif de propagation du mode optique $n_{eff}$ n'étant pas linéaire comme illustré sur la figure 6, il en résulte que la deuxième fibre optique de mesure 11 aura, selon la quantité d'hydrogène absorbée, une sensibilité différente.

Ainsi la deuxième fibre optique de mesure 11, en ayant une interaction avec l'hydrogène différente de celle de la première fibre optique de mesure 10, permet de fournir une mesure complémentaire à celle que fournit la première fibre optique de mesure 10.

Dans le cas où la deuxième fibre optique de mesure 11 présente une sensibilité différente de la première fibre optique de mesure 10, que ce soit par sa configuration ou par une pré-charge en hydrogène, le dispositif présente alors une plage sur laquelle l'hydrogène peut être détecté et/ou dosé qui est étendue en combinant les plages offertes par la première et la deuxième fibre optique de mesure 10, 11.

Dans le cas où la deuxième fibre optique de mesure 11 présente une réduction de la diffusion de l'hydrogène, cette différence induit une vitesse de diffusion réduite de l'hydrogène dans ladite deuxième fibre optique de mesure 11. Il en résulte un décalage dans la mesure entre la première et la deuxième fibre optique de mesure 10, 11 permettant de remonter à une information temporelle sur la source de d'hydrogène dans l'installation.

Bien entendu, selon ce troisième mode de réalisation, il est possible de combiner ces divers types de différence d'interaction entre la première et la deuxième fibre optique de mesure 10, 11 en les adaptant aux conditions d'installation du dispositif.

Il est également possible, selon un principe similaire à celui du troisième mode de réalisation, de combiner plus de deux fibres optiques de mesure de manière à permettre soit au dispositif de présenter une plus grande de plage sur laquelle l'hydrogène peut être détecté et/ou dosé, soit de combiner les avantages des différents types d'interaction avec l'hydrogène.

Bien entendu, un tel mode de réalisation, s'il est illustré sur la figure 11 pour une possibilité selon laquelle la configuration du dispositif est adaptée pour effectuer une mesure du type Brillouin selon le principe de la réflectométrie, présente également la possibilité, non illustrée, selon laquelle la configuration du dispositif 100 est adaptée pour effectuer une mesure du type Brillouin qui est réalisée par analyse dans le domaine temporel, fréquentiel ou de la corrélation. Selon cette possibilité, les première et seconde fibres optiques de mesure 10, 11 et la fibre optique de référence 30 peuvent être couplées au système optique parallèlement les unes des autres, en série ou toute combinaison hybride dans lesquelles deux fibres optiques parmi les première et seconde fibres optiques de mesure 10, 11 et la fibre optique de référence 30 sont mises en parallèle, la troisième fibre optique étant couplée individuellement au système optique 20.

Selon une possibilité non illustrée, il est également possible que la fibre optique de référence 10 soit adaptée pour effectuer une mesure d'autres paramètres que la température et/ou la déformation de la fibre optique, tels que la radioactivité présente dans l'installation, ceci sans que l'on sorte du cadre de l'invention. Une telle mesure peut permettre au dispositif de présenter une fonction de surveillance de cet autre paramètre en complément de sa fonction première qui est la détection et/ou le dosage de l'hydrogène dans l'installation à surveiller.

On peut également noter que chacun du moyen d'émission 21 et du moyen de mesure 22 peut comporter un ou plusieurs modules, un module pouvant être dédié à la mesure sur une seule fibre optique que celle-ci soit de référence ou de mesure, plusieurs fibres optiques ou l'ensemble des fibres optiques, cedi sans que l'on sorte du cadre de l'invention.

**Revendications**

1. Dispositif (100) de détection et/ou de dosage d'hydrogène destiné à la surveillance d'une installation (1), ledit dispositif (100) comportant :

   - une première fibre optique de mesure (10) destinée à équiper l'installation (1),
   - un système optique (20) relié optiquement à la première fibre optique de mesure (10) et adapté pour mesurer au moins un paramètre de la première fibre optique,

   le dispositif (100) **étant caractérisé en ce que** le système optique (20) est adapté pour effectuer la mesure du paramètre de la première fibre optique (10) le long de la première fibre optique de mesure (10) selon un principe de mesure du type Brillouin.

2. Dispositif (100) selon la revendication 1, dans lequel la mesure du paramètre de la première fibre optique est réalisée selon un principe de mesure du type Brillouin sélectionné dans le groupe comportant la mesure de réflectométrie optique Brillouin dans le domaine temporel, la mesure de réflectométrie optique Brillouin dans le domaine fréquentiel, la mesure de réflectométrie optique Brillouin dans le domaine de corrélation, la mesure optique Brillouin par analyse dans le domaine temporel, la mesure optique Brillouin dans le domaine fréquentiel et la mesure optique Brillouin par analyse dans le domaine de la corrélation.

3. Dispositif selon la revendication 1 ou 2, dans lequel le dispositif est adapté pour effectuer une mesure de référence d'au moins un paramètre de la première fibre optique de mesure (1) à au moins un emplacement de la première fibre optique de mesure (10), ladite mesure de référence n'étant pas influencée par la présence d'hydrogène

4. Dispositif (100) selon l'une quelconque des revendications précédentes, dans lequel le système optique (20) est adapté pour effectuer la mesure de référence le long de la première fibre optique à deux longueurs d'onde différentes.

5. Dispositif (100) selon la revendication 3 ou la revendication 1 ou 2 en combinaison avec la revendication 3, dans lequel la première fibre optique de mesure comporte au moins une portion rendue insensible à l'hydrogène de manière à ce que la mesure d'un paramètre de la première fibre optique de mesure (10) par le système optique (20) au niveau de la dite portion fournisse une mesure de référence.

6. Dispositif (100) selon la revendication 1 ou 2, dans lequel il est en outre prévu une fibre optique de référence (30) qui, étant destinée à équiper l'installation (1), est reliée au système optique (20), ladite fibre optique de référence (30) étant destinée à la fourniture d'une mesure de référence.

7. Dispositif (100) selon la revendication 6, dans lequel la fibre optique de référence (30) est configurée pour présenter une sensibilité réduite à l'hydrogène, préférentiellement pour être rendue insensible à l'hydrogène.

8. Dispositif (100) selon l'une quelconque des précédentes revendication dans laquelle il est en outre prévu une deuxième fibre optique de mesure (11) qui, étant destinée à équiper l'installation (1), est reliée au système optique (20), ladite deuxième fibre optique de mesure (11) étant configurée pour présenter une interaction avec l'hydrogène différente de celle de la première fibre optique de mesure (10).

9. Dispositif (100) selon la revendication 8, selon lequel la deuxième fibre optique de mesure (11) comporte un moyen de limitation de la diffusion d'hydrogène dans ladite deuxième fibre optique de mesure (11).

10. Dispositif (100) selon la revendication 8, selon lequel le moyen de limitation de la diffusion d'hydrogène de la deuxième fibre optique de mesure (11) comporte un revêtement de ladite deuxième fibre optique de mesure, ledit revêtement présentant une perméabilité partielle à l'hydrogène.

11. Dispositif (100) selon la revendication 8, selon lequel la deuxième fibre optique de mesure (11) présente une charge en hydrogène préalable configurée pour modifier l'interaction de ladite fibre optique (11) avec l'hydrogène.

12. Dispositif (100) selon la revendication 5 ou 6 ou l'une quelconque des revendications 7 à 10 en combinaison avec la revendication 5 ou 6, dans lequel la ou les fibres optiques de mesure (10, 11) et la fibre optique de référence (30) sont placées sous la forme d'un faisceau ou d'une nappe.

13. Procédé de détection et/ou dosage d'hydrogène, pour surveiller une installation, ledit procédé comportant les étapes consistant à :

- installer une première fibre optique de mesure dans l'installation,
- réaliser une mesure d'un paramètre le long de la fibre optique de mesure selon le principe de la mesure du type Brillouin,
- respectivement détecter et/ou doser l'hydrogène dans la première fibre optique de mesure (10) à partir du paramètre mesuré le long de la fibre optique de mesure selon le principe de la mesure du type Brillouin.

14. Procédé de détection et/ou de dosage d'hydrogène **caractérisé en ce qu'**il met en oeuvre un dispositif (100) selon l'une quelconque des précédentes revendications 1 à 12 et ce qu'il comporte les étapes consistant à :

- mettre en oeuvre le système optique (20) pour mesurer un paramètre de la première fibre optique de mesure le long de la première fibre optique de mesure selon le principe de la mesure du type Brillouin,
- respectivement détecter et/ou doser l'hydrogène dans la première fibre optique de mesure (10) à partir du paramètre mesuré le long de la fibre optique de mesure selon le principe de la mesure du type Brillouin.

15. Procédé selon la la revendication 14, dans lequel il est mis en oeuvre un dispositif selon la revendication 3 ou 6, ou l'une quelconque des revendication 4 à 11 en combinaison avec la revendication 3 ou 6, l'étape respective de détection et/ou dosage comportant les sous-étapes consistant à :

- mettre en oeuvre le système optique (20) pour effectuer une mesure de référence le long de la première fibre optique de référence (10),
- corriger sur la base de la mesure de référence la mesure du paramètre de la première fibre optique de mesure (10) effectuée le long de la première fibre optique de mesure (10) selon le principe de la mesure du type Brillouin,
- respectivement détecter et/ou mesurer la présence d'hydrogène dans la première fibre optique de mesure (10) à partir de la mesure du paramètre de la première fibre optique de mesure (10) le long de la première fibre optique de mesure (10) selon le principe de la mesure du type Brillouin qui a été corrigée à partir de la mesure de référence.

**Patentansprüche**

1. Vorrichtung (100) zur Erfassung und/oder Dosierung von Wasserstoff für die Überwachung einer Anlage (1), wobei die Vorrichtung (100) umfasst:

- eine erste optische Messfaser (10), die dazu ausgelegt ist, zur Ausstattung der Anlage (1) zu gehören,
- ein optisches System (20), das optisch mit der ersten optischen Messfaser (10) verbunden und dazu ausgelegt ist, wenigstens einen Parameter der ersten optischen Faser zu messen,

wobei die Vorrichtung (100) **dadurch gekennzeichnet ist, dass** das optische System (20) dazu ausgelegt ist, die Messung des Parameters der ersten optischen Faser (10) entlang der ersten optischen Messfaser (10) gemäß einem Messprinzip vom Brillouin-Typ durchzuführen.

2. Vorrichtung (100) nach Anspruch 1, bei der die Messung des Parameters der ersten optischen Faser gemäß einem Messprinzip vom Brillouin-Typ durchgeführt wird, ausgewählt aus der Gruppe umfassend die Messung der optischen Brillouin-Reflektometrie in der Zeitdomäne, die Messung der optischen Brillouin-Reflektometrie in der Frequenzdomäne, die Messung der optischen Brillouin-Reflektometrie in der Korrelationsdomäne, die optische Brillouin-Messung durch Analyse in der Zeitdomäne, die optische Brillouin-Messung in der Frequenzdomäne und die optische Brillouin-Messung durch Analyse in der Korrelationsdomäne.

3. Vorrichtung nach Anspruch 1 oder 2, bei der die Vorrichtung dazu ausgelegt ist, eine Referenzmessung wenigstens eines Parameters der ersten optischen Messfaser (1) an wenigstens einer Stelle der ersten optischen Messfaser (10) durchzuführen, wobei die Referenzmessung durch die Anwesenheit von Wasserstoff nicht beeinflusst wird.

4. Vorrichtung (100) nach einem der vorhergehenden Ansprüche, bei der das optische System (20) dazu ausgelegt ist, die Referenzmessung entlang der ersten optischen Faser bei zwei verschiedenen Wellenlängen durchzuführen.

5. Vorrichtung (100) nach Anspruch 3 oder Anspruch 1 oder 2 in Kombination mit Anspruch 3, bei der die erste optische Messfaser wenigstens einen Bereich umfasst, der derart für Wasserstoff unempfindlich gemacht ist, dass die Messung eines Parameters der ersten optischen Messfaser (10) durch das optische System (20) auf Höhe des Bereichs eine Referenzmessung liefert.

**6.** Vorrichtung (100) nach Anspruch 1 oder 2, bei der ferner eine optische Referenzfaser (30) vorgesehen ist, die dazu ausgelegt ist, zur Ausstattung der Anlage (1) zu gehören, und die mit dem optischen System (20) verbunden ist, wobei die optische Referenzfaser (30) zum Liefern einer Referenzmessung ausgelegt ist.

**7.** Vorrichtung (100) nach Anspruch 6, bei der die optische Referenzfaser (30) dazu ausgelegt ist, eine verringerte Empfindlichkeit für Wasserstoff aufzuweisen, vorzugsweise für Wasserstoff unempfindlich gemacht zu sein.

**8.** Vorrichtung (100) nach einem der vorhergehenden Ansprüche, bei der ferner eine zweite optische Messfaser (11) vorgesehen ist, die dazu ausgelegt ist, zur Ausstattung der Anlage (1) zu gehören und mit dem optischen System (20) verbunden ist, wobei die zweite optische Messfaser (11) dazu ausgelegt ist, eine Wechselwirkung mit Wasserstoff aufzuweisen, die verschieden ist von jener der ersten optischen Messfaser (10).

**9.** Vorrichtung (100) nach Anspruch 8, bei der die zweite optische Messfaser (11) eine Einrichtung zur Begrenzung der Diffusion von Wasserstoff in der zweiten optischen Messfaser (11) umfasst.

**10.** Vorrichtung (100) nach Anspruch 8, bei der die Einrichtung zur Begrenzung der Diffusion von Wasserstoff der zweiten optischen Messfaser (11) eine Beschichtung der zweiten optischen Messfaser umfasst, welche Beschichtung eine partielle Permeabilität für Wasserstoff aufweist.

**11.** Vorrichtung (100) nach Anspruch 8, bei der die zweite optische Messfaser (11) eine zuvor konfigurierte Wasserstoffbeladung aufweist, um die Wechselwirkung der optischen Faser (11) mit Wasserstoff zu modifizieren.

**12.** Vorrichtung (100) nach Anspruch 5 oder 6 oder einem der Ansprüche 7 bis 10 in Kombination mit Anspruch 5 oder 6, bei der die optische(n) Messfaser(n) (10, 11) sowie die optische Referenzfaser (30) in Form eines Strahls oder einer Ebene platziert sind.

**13.** Verfahren zur Erfassung und/oder Dosierung von Wasserstoff zur Überwachung einer Anlage, welches Verfahren die folgenden Schritte umfasst:

- Installieren einer ersten optischen Messfaser in der Anlage,
- Durchführen einer Messung eines Parameters entlang der optischen Messfaser gemäß dem Messprinzip vom Brillouin-Typ,
- jeweils Erfassen und/oder Dosieren von Wasserstoff in der ersten optischen Messfaser (10) auf Grundlage des Parameters, der entlang der optischen Messfaser gemäß dem Messprinzip vom Brillouin-Typ gemessen wird.

**14.** Verfahren zur Erfassung und/oder Dosierung von Wasserstoff, **dadurch gekennzeichnet, dass** es eine Vorrichtung (100) nach einem der vorhergehenden Ansprüche 1 bis 12 verwendet, und die folgenden Schritte umfasst:

- Verwenden des optischen Systems (20) zum Messen eines Parameters der ersten optischen Messfaser entlang der ersten optischen Messfaser gemäß dem Messprinzip vom Brillouin-Typ,
- jeweils Erfassung und/oder Dosieren von Wasserstoff in der ersten optischen Messfaser (10) ausgehend von dem Parameter, der entlang der optischen Messfaser gemäß dem Messprinzip vom Brillouin-Typ gemessen wird.

**15.** Verfahren nach Anspruch 14, bei dem eine Vorrichtung nach Anspruch 3 oder 6 oder einem der Ansprüche 4 bis 11 in Kombination mit Anspruch 3 oder 6 verwendet wird, wobei der jeweilige Schritt der Erfassung und/oder Dosierung die folgenden Unterschritte umfasst:

- Verwenden des optischen Systems (20) zum Durchführen einer Referenzmessung entlang der ersten optischen Referenzfaser (10),
- Korrigieren, auf Basis der Referenzmessung, der Messung des Parameters der ersten optischen Messfaser (10), die entlang der ersten optischen Messfaser (10) gemäß dem Messprinzip vom Brillouin-Typ durchgeführt wird,
- jeweils Erfassen und/oder Messen der Anwesenheit von Wasserstoff in der ersten optischen Messfaser (10) ausgehend von der Messung des Parameters der ersten optischen Messfaser (10) entlang der ersten optischen Messfaser (10) gemäß dem Messprinzip vom Brillouin-Typ, die ausgehend von der Referenzmessung korrigiert wurde.

**Claims**

**1.** Device (100) for the detection and/or quantitative analysis of hydrogen intended for the monitoring of an installation (1), said device (10) comprising:

- a first measuring optical fibre (10) intended to equip the installation (1),
- an optical system (20) optically connected to the first measuring optical fibre (10) and suitable for measuring at least one parameter of the first optical fibre,

the device (100) being **characterised in that** the optical system (20) is suitable for measuring the parameter of the first optical fibre (10) along the first measuring optical fibre (10) according to a measurement principle of the Brillouin type.

2. Device (100) according to claim 1, in which the measurement of the parameter of the first optical fibre is measured according to a Brillouin measurement principle selected from the group comprising Brillouin optical reflectometry measurement in the time domain, Brillouin optical reflectometry measurement in the frequency domain, Brillouin optical reflectometry measurement in the correlation domain, Brillouin optical measurement by analysis in the time domain, Brillouin optical measurement in the frequency domain and Brillouin optical measurement by analysis in the correlation domain.

3. Device according to claim 1 or 2, in which the device is adapted to make a reference measurement of at least one parameter of the first measuring optical fibre (1) at at least one location on the first measuring optical fibre (10), said reference measurement not being influenced by the presence of hydrogen.

4. Device (100) according to any one of the preceding claims, in which the optical system (20) is adapted to make the reference measurement along the first optical fibre at two different wavelengths.

5. Device (100) according to claim 3 or claim 1 or 2 in combination with claim 3, in which the first measuring optical fibre comprises at least one portion made insensitive to hydrogen so that the measurement of a parameter of the first measuring optical fibre (10) by the optical system (20) at said portion supplies a reference measurement.

6. Device (100) according to claim 1 or 2, in which a reference optical fibre (30) is also provided which, being intended to equip the installation (1), is connected to the optical system (20), said reference optical fibre (30) being intended to supply a reference measurement.

7. Device (100) according to claim 6, in which the reference optical fibre (30) is configured so as to have reduced sensitivity to hydrogen, preferably so as to be made insensitive to hydrogen.

8. Device (100) according to any one of the preceding claims, in which a second measuring optical fibre (11) is also provided which, being intended to equip the installation (1), is connected to the optical system (20), said second measuring optical fibre (11) being configured so as to have an interaction with hydrogen different from that of the first measuring optical fibre (10).

9. Device (100) according to claim 8, according to which the second measuring optical fibre (11) comprises a means for limiting the diffusion of hydrogen in said second measuring optical fibre (11).

10. Device (100) according to claim 8, according to which the means for limiting the diffusion of hydrogen of the second measuring optical fibre (11) comprises a cladding of said second measuring optical fibre, said cladding have partial permeability to hydrogen.

11. Device (100) according to claim 8, according to which the second measuring optical fibre (11) has a prior hydrogen load configured so as to modify the interaction of said optical fibre (11) with hydrogen.

12. Device (100) according to claim 5 or 6 or any one of claims 7 to 10 in combination with claim 5 or 6, in which the optical measuring fibre or fibres (10, 11) and the reference optical fibre (30) are placed in the form of a cluster or ribbon.

13. Method for the detection and/or quantitative analysis of hydrogen, for monitoring an installation, said method comprising the steps consisting of:

   - installing a first measuring optical fibre in the installation,
   - making a measurement of a parameter along the measuring optical fibre according to the principle of Brillouin measurement,
   - respectively detecting and/or quantitatively analysing the hydrogen in the measurement optical fibre (10) from the parameter measured along the measuring optical fibre according to the principle of Brillouin measurement.

14. Method for the detection and/or quantitative analysis of hydrogen **characterised in that** it uses a device (100) according to any one of the preceding claims 1 to 12 and comprising the steps consisting of:

   - using the optical system (20) for measuring a parameter of the first measuring optical fibre along the first measuring optical fibre according to the principle of Brillouin measurement,
   - respectively detecting and/or quantitatively analysing the hydrogen in the first measuring optical fibre (10) from the parameter measured along the measuring optical fibre according to the principle of Brillouin measurement.

15. Method according to claim 14, in which a device according to claim 3 or 6, or any one of claims 4 to 11 in combination with claim 3 or 6, is used, the respective detection and/or quantitative analysis step comprising the sub-steps consisting of:

- using the optical system (20) for making a reference measurement along the first measuring optical fibre (10),
- correcting, on the basis of the reference measurement, the measurement of the parameter of the first measuring optical fibre (10) made along the first measuring optical fibre (10) according to the principle of Brillouin measurement,
- respectively detecting and/or measuring the presence of hydrogen in the first measuring optical fibre (10) from the measurement of the parameter of the first measuring optical fibre (10) along the first measuring optical fibre (10) according to the principle of Brillouin measurement that was corrected using the reference measurement.

FIG. 1

FIG. 2

FIG. 3

FIG. 4

FIG. 5

FIG. 6

FIG. 7

FIG. 8

FIG. 9

FIG. 10

FIG. 11

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2009067671 A
- WO 2008136870 A
- JP 61017048 A

- WO 2011115686 A
- EP 1195628 A
- EP 1426804 A